# EUROPÄISCHE PATENTANMELDUNG

(11) **EP 4 740 844 A1**
(43) Veröffentlichungstag der Anmeldung: **13.05.2026**
(21) Anmeldenummer: 25213001.8
(22) Anmeldetag: 03.11.2025
(51) Int. Cl.: A61B 5/00

(54) **MEDIZINISCHE LICHTQUELLENVORRICHTUNG, MEDIZINISCHES BILDGEBUNGSSYSTEM UND VERFAHREN ZUM BETRIEB EINER MEDIZINISCHEN LICHTQUELLENVORRICHTUNG UND/ODER EINES MEDIZINISCHEN BILDGEBUNGSSYSTEMS**

(30) Priorität: 06.11.2024 DE 102024132318
(71) Anmelder: Karl Storz SE & Co. KG, 78532 Tuttlingen (DE)
(72) Erfinder: Buschle, Lukas, 78532 Tuttlingen (DE)

(57) **Zusammenfassung**

Die vorliegende Erfindung betrifft eine medizinische Lichtquellenvorrichtung (10), umfassend ein Beleuchtungsmittel (12), welches dazu eingerichtet ist, Licht mit einem Ausgangslichtspektrum (92) zu erzeugen, und ein Konvertierungselement (16), welches dazu eingerichtet ist, das Licht mit dem Ausgangslichtspektrum (92) zumindest teilweise in Licht mit einem zweiten Lichtspektrum (18) derart zu wandeln, dass Beleuchtungslicht gleichzeitig mit Licht mit einem ersten Lichtspektrum (14) und mit dem zweiten Lichtspektrum (18) bereitstellbar ist, wobei das erste Lichtspektrum (14) spektral dem Ausgangslichtspektrum (92) entspricht, wobei das erste Lichtspektrum (14) zumindest teilweise in einem kurzwelligeren Wellenlängenbereich als das zweite Lichtspektrum (18) liegt, wobei das erste Lichtspektrum (14) das zweite Lichtspektrum (18) höchstens zu 50 % überlappt, wobei das erste Lichtspektrum (14) im Vergleich zu dem zweiten Lichtspektrum (18) eine geringere Bandbreite (20) aufweist, und wobei anhand des ersten Lichtspektrums (14) und des zweiten Lichtspektrums (18) wenigstens ein physiologischer Parameter bestimmbar ist.

## Beschreibung

Die vorliegende Anmeldung betrifft eine medizinische Lichtquellenvorrichtung, ein medizinisches Bildgebungssystem und ein Verfahren zum Betrieb einer medizinischen Lichtquellenvorrichtung und/oder eines medizinischen Bildgebungssystems.

Aus dem Stand der Technik sind Vorrichtungen und Verfahren zur multispektralen Bildgebung bekannt. Bei der multispektralen Bildgebung wird ausgenutzt, dass ein Objekt wellenlängenabhängige optische Eigenschaften haben kann. Um diese sichtbar zu machen, wird bei der sogenannten lichtquellenseitigen multispektralen Bildgebung das Objekt mit Beleuchtungslicht in unterschiedlichen Wellenlängenbereichen beleuchtet. So können dann für die Wellenlängenbereiche spezifische Bilder des Objekts erfasst sowie wellenlängenspezifische Bildinformationen erzeugt und ausgewertet werden. Dadurch können in weiterer Folge Parameter berechnet werden, anhand derer eine Veränderung oder ein Zustand des Objekts abgeschätzt werden kann. Gerade im medizinischen Bereich gewinnt multispektrale Bildgebung zunehmend an Bedeutung, da sich durch sie etwa eine Oxygenierung eines Gewebes oder das Vorhandensein eines Tumors effizient feststellen lassen.

Bei der lichtquellenseitigen multispektralen Bildgebung sind nach dem Stand der Technik mehrere Beleuchtungsmittel erforderlich, die jeweils Licht mit einem unterschiedlichen Lichtspektrum bereitstellen. Die Wellenlängenbereiche dieser Lichtspektren sind dabei zueinander verschoben. Beispielsweise können ein rotes und ein grünes Beleuchtungsmittel verwendet werden, deren emittiertes Licht auf einen gemeinsamen Pfad geführt wird. Zur multispektralen Bildgebung werden dann die beiden Beleuchtungsmittel nacheinander aktiviert und jeweils ein Bild des Objekts aufgenommen. Zusätzlich ist üblicherweise noch eine Weißlicht-Beleuchtungsmittel vorgesehen, damit neben Multispektralbildern auch Weißlichtbilder aufgenommen werden können.

Wie beschrieben, erfordert der lichtquellenseitige Aufbau die Verwendung mehrerer Beleuchtungsmittel, was zu einer Reihe von Herausforderungen führt. Zum einen nimmt die Anzahl an medizinischen Geräten mit dem technologischen Fortschritt in einem Operationssaal rapide zu. Der Raumbedarf für die medizinischen Geräte steigt also immer weiter an.

Zum anderen erfordert die Verwendung mehrerer Beleuchtungsmittel eine sorgfältige Abstimmung und Kalibrierung der räumlichen Verteilung des emittierten Lichts, um eine gleichmäßige und präzise Beleuchtung zu gewährleisten. Diese Kalibrierung ist technisch anspruchsvoll und zeitaufwendig, was die Produktionskosten erhöht und die Zuverlässigkeit der Systeme beeinträchtigen kann.

Außerdem wird das Wärmemanagement einer Lichtquellenvorrichtung für die lichtquellenseitige multispektrale Bildgebung aufwendig und konstruktiv herausfordernd. Üblicherweise sind die einzelnen Beleuchtungsmittel auf eigenen Kühleinrichtungen wie Kühlkörpern angeordnet, damit ein thermisch stabiler Betrieb in einem engen thermischen Betriebsfenster möglich ist. Innerhalb des Betriebsfensters emittiert das Beleuchtungsmittel Licht mit einer vorbestimmten, kalibrierten Intensität, wodurch gewünschte Beleuchtungsintensitäten erreicht und in weiterer Folge reproduzierbare Multispektralbilder möglich werden. Da sich die einzelnen Beleuchtungsmittel thermisch gegenseitig beeinflussen können, müssen die Beleuchtungsmittel gezielt und überlegt angeordnet werden. Diese Anordnung wird mit jedem weiteren vorgesehenem Beleuchtungsmittel überproportional komplexer.

Ein weiterer Aspekt betrifft Endoskope mit distalseitig verbauten Bildsensoren und Beleuchtungsmitteln. Derart verbaute Bildsensoren und Beleuchtungsmittel sind bei flexiblen Endoskopen und auch bei Stereoendoskopen verbreitet. Bei solchen Endoskopen ist der verfügbare Bauraum im Schaft des Endoskops begrenzt, was eine Integration zusätzlicher Elemente konstruktiv herausfordernd macht.

Ausgehend vom Stand der Technik kann der Erfindung die Aufgabe zugrunde liegen, effizient Beleuchtungslicht für die multispektrale Bildgebung bereitzustellen.

Die Aufgabe wird erfindungsgemäß durch eine medizinische Lichtquellenvorrichtung, ein medizinisches Bildgebungssystem und Verfahren zum Betrieb einer medizinischen Lichtquellenvorrichtung und/oder eines medizinischen Bildgebungssystems gelöst, wie sie hierin beschrieben und in den Ansprüchen definiert sind.

Die vorliegende Erfindung sieht vor, eine medizinische Lichtquellenvorrichtung bereitzustellen. Diese umfasst ein Beleuchtungsmittel, welches dazu eingerichtet ist, Licht mit einem Ausgangslichtspektrum zu erzeugen. Zudem umfasst die Lichtquellenvorrichtung ein Konvertierungselement, welches dazu eingerichtet ist, das Licht mit dem Ausgangslichtspektrum zumindest teilweise in Licht mit einem zweiten Lichtspektrum derart zu wandeln, dass Beleuchtungslicht gleichzeitig mit Licht mit einem ersten Lichtspektrum und dem zweiten Lichtspektrum bereitstellbar ist, insbesondere in zumindest einem Betriebszustand bereitgestellt wird. Dabei entspricht das erste Lichtspektrum spektral dem Ausgangslichtspektrum. Das erste Lichtspektrum liegt zumindest teilweise in einem kurzwelligeren Wellenlängenbereich als das zweite Lichtspektrum. Zudem überlappt das erste Lichtspektrum das zweite Lichtspektrum zu höchstens 50%. Das erste Lichtspektrum weist außerdem im Vergleich zu dem zweiten Lichtspektrum eine geringere Bandbreite auf. Anhand des ersten Lichtspektrums und des zweiten Lichtspektrums ist wenigstens ein physiologischer Parameter bestimmbar.

Die Erfindung sieht zudem vor, ein medizinisches Bildgebungssystem bereitzustellen, insbesondere ein endoskopisches, mikroskopisches und/oder exoskopisches Bildgebungssystem. Das Bildgebungssystem umfasst eine erfindungsgemäße medizinische Lichtquellenvorrichtung, welche dazu eingerichtet ist, Beleuchtungslicht zur Beleuchtung eines Objektbereichs bereitzustellen. Zudem umfasst das Bildgebungssystem eine Bilderfassungseinrichtung, welche dazu eingerichtet ist, den Objektbereich abzubilden und Bilddaten des Objektbereichs zu erzeugen. Die Bilddaten umfassen räumliche und spektrale Informationen, welche sich auf eine Rückstrahlung des Objektbereichs in Folge der Beleuchtung mit Licht mit dem ersten Lichtspektrum und dem zweiten Lichtspektrum beziehen.

Zudem sieht die vorliegende Erfindung vor, ein Verfahren zum Betrieb einer medizinischen Lichtquellenvorrichtung, insbesondere einer erfindungsgemäßen Lichtquellenvorrichtung, und/oder eines medizinischen Bildgebungssystems, insbesondere eines erfindungsgemäßen Bildgebungssystems, bereitzustellen. Das Verfahren umfasst die folgenden Schritte:
- Erzeugen von Licht mit einem Ausgangslichtspektrum; und
- Wandeln des Lichts mit dem Ausgangslichtspektrum zumindest teilweise in Licht mit einem zweiten Lichtspektrum derart, dass Beleuchtungslicht gleichzeitig mit Licht mit einem ersten Lichtspektrum und mit dem zweiten Lichtspektrum bereitstellbar ist.

Das erste Lichtspektrum entspricht dabei spektral dem Ausgangslichtspektrum. Das erste Lichtspektrum liegt zumindest teilweise in einem kurzwelligeren Wellenlängenbereich als das zweite Lichtspektrum. Zudem überlappt das erste Lichtspektrum das zweite Lichtspektrum höchstens zu 50 % überlappt und das erste Lichtspektrum weist im Vergleich zu dem zweiten Lichtspektrum eine geringere Bandbreite auf. Außerdem ist anhand des ersten Lichtspektrums und des zweiten Lichtspektrums wenigstens ein physiologischer Parameter bestimmbar.

Die erfindungsgemäßen Merkmale erlauben es, effizient Beleuchtungslicht für die multispektrale Bildgebung bereitzustellen. Es werden erfindungsgemäß weniger Beleuchtungsmittel benötigt, um Multispektralbildgebung durchzuführen. In anderen Worten kann ein spektraler Arbeitsbereich, insbesondere für die Multispektralbildgebung, erweitert werden, ohne eine größere Anzahl an Beleuchtungsmitteln vorzusehen. Soll der Objektbereich beispielsweise mit Licht in zwei unterschiedlichen Wellenlängenbereichen bzw. zwei verschiedenen kombinierten Lichtspektren beleuchtet werden, reicht ein Beleuchtungsmittel aus, wenn das Konvertierungselement verwendet wird. Die Lichtquellenvorrichtung kann also eine geringere Anzahl an Elementen aufweisen, wodurch sie kompakter ausgebildet sein kann. Es kann etwa eine Lichtquellenvorrichtung bereitgestellt werden, die in etwa dieselbe räumliche Ausdehnung wie eine Lichtquellenvorrichtung nach dem Stand der Technik hat, zusätzlich jedoch für die Multispektralbildgebung geeignet ist, bzw. dazu eingerichtet ist, Beleuchtungslicht mit mehreren unterschiedlichen Lichtspektren bereitzustellen.

Durch die erfindungsgemäßen Merkmale kann zudem das Wärmemanagement einfacher durchführbar sein und die Beleuchtungsmittel durch konstruktiv einfache Maßnahmen in dem Betriebsfenster betrieben werden. Beispielsweise kann die Lichtquellenvorrichtung mit einer bekannten Kühlvorrichtung betrieben werden, wobei der spektrale Arbeitsbereich jedoch erweitert sein kann. Es kann also eine kompakte Lichtquellenvorrichtung durch einfache konstruktive Maßnahmen bereitgestellt werden, mittels derer der physiologische Parameter bestimmt werden kann. Zur Bestimmung des physiologischen Parameters kann spezifisch für das erste Lichtspektrum und das zweite Lichtspektrum ein Bild des Objektbereichs erzeugt werden. Der Objektbereich kann dadurch unter Verwendung der Lichtquellenvorrichtung hinsichtlich physiologischer Eigenschaften effizient untersucht werden.

Die medizinische Lichtquellenvorrichtung kann etwa eine tragbare Lichtquellenvorrichtung umfassen, die beispielsweise modulhaft ausgebildet ist. Diese kann separat in einem Operationssaal oder dergleichen vorgesehen werden. Eine solche Lichtquellenvorrichtung kann beispielsweise über ein eigenes Gehäuse verfügen, welches das Beleuchtungsmittel und das Konvertierungselement einhaust. In anderen Ausführungsformen kann die medizinische Lichtquellenvorrichtung in eine bestehende Vorrichtung wie beispielsweise ein Endoskop integriert sein.

Das Beleuchtungsmittel kann eine Leuchtdiode (LED), einen Diodenlaser, einen Gaslaser, eine Spektrallampe, beispielsweise eine Xenonlampe, und/oder dergleichen umfassen, welches insbesondere innerhalb des Gehäuses angeordnet sein kann. Alternativ kann das Beleuchtungsmittel etwa an einer distalen Endfläche eines Endoskopschafts angeordnet sein. Das Beleuchtungsmittel kann ein optisches Element aufweisen, das dazu eingerichtet ist, emittiertes Licht spektral zu beeinflussen. Mittels des optischen Elements kann ein Intensitätsverlauf des ersten Lichtspektrums gezielt und/oder anwendungsspezifisch vorgesehen werden.

Unter einem Lichtspektrum kann hierin eine Intensitätsverteilung über einen bestimmten dem Lichtspektrum zugeordneten Wellenlängenbereich verstanden werden. Das Ausgangslichtspektrum kann einen Ausgangswellenlängenbereich aufweisen und das erste Lichtspektrum entsprechend einen ersten Wellenlängenbereich aufweisen. Ebenso kann das zweite Lichtspektrum einen zweiten Wellenlängenbereich aufweisen. Der Ausgangswellenlängenbereich kann dem ersten Wellenlängenbereich entsprechen. In dem entsprechenden Wellenlängenbereich kann ein Großteil der Intensität des Lichts vorliegen, bzw. sich die Intensität zu einem Großteil innerhalb des zugeordneten Wellenlängenbereichs verteilen. Unter einem "Großteil" kann beispielsweise zumindest 90 %, insbesondere zumindest 95%, bevorzugt zumindest 98 % verstanden werden. Die Lichtintensität des ersten Lichtspektrums kann also zu einem Großteil auf den ersten Wellenlängenbereich verteilt sein. Entsprechendes gilt für die Lichtintensität des zweiten Lichtspektrums und/oder der Ausgangslichtspektrums. Es versteht sich, dass sich außerhalb des Wellenlängenbereichs geringfügige Lichtanteile befinden können. Diese können hinsichtlich der Wechselwirkung mit dem Objektbereich und/oder der Bildgebung vernachlässigbar klein sein. Weist ein Lichtspektrum in anderen Worten einen Wellenlängenbereich von A bis B auf, können sich unterhalb respektive oberhalb dieser Grenzen Lichtanteile befinden.

Da der Ausgangswellenlängenbereich dem ersten Wellenlängenbereich entsprechen kann, beziehungsweise das erste Lichtspektrum spektral dem Ausgangslichtspektrum entspricht, kann ein Teil des durch das Beleuchtungsmittel erzeugten Lichts ohne eine spektrale Wandlung zur Beleuchtung verwendet werden. Das Ausgangslichtspektrum kann sich dabei durch das teilweise Wandeln in das zweite Lichtspektrum bezüglich seiner Intensität verändern. Das erste Lichtspektrum kann einem abgeschwächten Ausgangslichtspektrum entsprechen.

Das Beleuchtungsmittel kann dazu eingerichtet sein, das Licht mit dem Ausgangslichtspektrum entlang seiner optischen Achse zu emittieren. Die optische Achse kann sich senkrecht von einer Emissionsfläche des Beleuchtungsmittels erstrecken. Das Konvertierungselement kann in der optischen Achse angeordnet sein, insbesondere derart, dass die optische Achse das Konvertierungselement schneidet. Das Licht kann dadurch entlang der optischen Achse gewandelt werden.

Das Konvertierungselement kann ein optisches Element umfassen, das dazu eingerichtet ist, in Wechselwirkung mit dem Licht mit dem Ausgangslichtspektrum zu treten. Es kann etwa bestimmt vorgesehene Absorptionseigenschaften in dem Ausgangswellenlängenbereich des Ausgangslichtspektrums aufweisen. In einigen Ausführungsformen kann das Konvertierungselement ein Phosphor-Konvertierungsmaterial, auch "Phosphor" genannt, umfassen. Beispiele für das Phosphor-Konvertierungsmaterial sind Yttrium-Aluminium-Granat, Silikat-Phosphore, Nitrid-Phosphore, seltenerdmetall-dotierte Phosphore und/oder dergleichen. Das Konvertierungselement kann etwa in Form eines Phosphorplättchens ausgebildet sein. Denkbar ist auch der Einsatz von Quantumdots zur Wandlung des Lichts mit dem Ausgangslichtspektrum in Licht mit dem zweiten Lichtspektrum. Ebenso kann das Konvertierungselement eine Beschichtung auf dem Beleuchtungsmittel umfassen.

Ferner kann das Konvertierungselement derart lichtdurchlässig sein, insbesondere teildurchlässig, dass ein Anteil des Lichts mit dem Ausgangslichtspektrum durch das Konvertierungselement hindurchtritt. Dieser Anteil kann spezifisch für eine bestimmte Anwendung vorgesehen werden und/oder das Licht mit dem ersten Lichtspektrum definieren. Das erste Lichtspektrum kann entsprechend und wie bereits erläutert einem abgeschwächten Ausgangslichtspektrum entsprechen.

Gemäß einigen Ausführungsformen wird das Beleuchtungslicht gleichzeitig mit Licht mit dem ersten Lichtspektrum und dem zweiten Lichtspektrum bereitgestellt. Gemäß anderen Ausführungsformen kann gezielt ein Betriebszustand eingestellt werden, in dem das Beleuchtungslicht gleichzeitig mit Licht mit dem ersten Lichtspektrum und dem zweiten Lichtspektrum bereitgestellt wird. In diesen Ausführungsformen wird derartiges Beleuchtungslicht also wahlweise bereitgestellt. Wahlweise kann auch Beleuchtungslicht bereitstellbar sein, das Licht mit dem Ausgangslichtspektrum umfasst. Beispielsweise kann das Konvertierungselement mittels einer geeigneten Einrichtung in den optischen Pfad des Beleuchtungsmittels gefahren werden. Dies kann in Art eines Einschiebens eines optischen Filters geschehen, beispielsweise mittels eines Filterrevolvers und/oder dergleichen, der das Konvertierungselement trägt. Das Konvertierungselement kann also in einem, insbesondere ersten, Betriebszustand in dem optischen Pfad angeordnet sein. In diesem Betriebszustand wird das Beleuchtungslicht gleichzeitig mit Licht mit dem ersten Lichtspektrum und dem zweiten Lichtspektrum bereitgestellt. In einem weiteren, insbesondere zweiten, Betriebszustand kann das Konvertierungselement außerhalb des optischen Pfads angeordnet sein, insbesondere derart, dass es das Licht mit dem Ausgangslichtspektrum nicht wandelt. Die Lichtquellenvorrichtung kann also dazu eingerichtet sein, das Beleuchtungslicht gleichzeitig mit Licht mit dem ersten Lichtspektrum und dem zweiten Lichtspektrum wahlweise bereitzustellen.

Unter "Beleuchtungslicht" kann Licht verstanden werden, das an einem Ausgang bzw. einer optischen Schnittstelle der Lichtquellenvorrichtung abgreifbar ist und/oder aus der Lichtquellenvorrichtung ausgekoppelt wird. Beispielsweise könnte die Lichtquellenvorrichtung eine Linse umfassen, mittels derer Licht ausgekoppelt werden kann. Unter dem durch diese Linse ausgekoppelten Licht kann das Beleuchtungslicht verstanden werden. Dieses Licht ist also jenes, das zur Beleuchtung des Objektbereichs verwendet werden kann. Es versteht sich, dass das Beleuchtungslicht außerhalb der Lichtquellenvorrichtung Änderungen hinsichtlich spektraler Eigenschaften, der Intensität und/oder dergleichen erfahren kann.

Ferner kann das Konvertierungselement dazu eingerichtet sein, einfallendes Licht zumindest teilweise in Licht mit einer längeren Wellenlänge zu wandeln. Entsprechend kann das zweite Lichtspektrum einen zweiten Wellenlängenbereich aufweisen, der größere Wellenlängen umfasst als der erste Wellenlängenbereich des ersten Lichtspektrums, bzw. als der Ausgangswellenlängenbereich des Ausgangslichtspektrums. Das zweite Lichtspektrum kann spektral zu größeren Wellenlängen verschoben sein. In anderen Worten kann die mittlere Lichtintensität des ersten Lichtspektrums bei einer kleineren Wellenlänge liegen als die mittlere Lichtintensität des zweiten Lichtspektrums.

Ferner kann bei der Wandlung des Lichts mit dem Ausgangslichtspektrum zu Licht mit dem zweiten Lichtspektrum der Wellenlängenbereich verbreitert werden. In einigen Ausführungsformen verteilt sich die Intensität des zweiten Lichtspektrums über einen breiteren Wellenlängenbereich als das Ausgangslichtspektrum und/oder das erste Lichtspektrum. Das erste Lichtspektrum weist also eine geringere Bandbreite auf als das zweite Lichtspektrum.

Unter einer Gesamtintensitätsverteilung umfassend Licht mit dem ersten und dem zweiten Lichtspektrum kann ein Gesamtlichtspektrum des Beleuchtungslichts verstanden werden. Die Gesamtintensitätsverteilung bzw. das Gesamtlichtspektrum des Beleuchtungslichts umfassend Licht mit dem ersten und dem zweiten Lichtspektrum kann in einem Wellenlängenbereich zwischen dem ersten Lichtspektrum und dem zweiten Lichtspektrum und/oder zwischen zwei Mittelwellenlängen, die jeweils bei einem Intensitätsmittelwert des ersten respektive des zweiten Lichtspektrums liegen, ein Intensitätstal bzw. ein lokales Intensitätsminimum aufweisen. Das erste Lichtspektrum und das zweite Lichtspektrum sind dadurch voneinander getrennt und weisen jeweils eine eigene spezifische Intensitätsverteilung auf. Es ist zwar denkbar, dass das erste Lichtspektrum fließend in das zweite Lichtspektrum übergehen kann, in einem entsprechenden Übergangsbereich vom ersten Lichtspektrum zum zweiten Lichtspektrum kann die Intensität beispielsweise maximal 30 %, insbesondere maximal 20 %, bevorzugt maximal 10 %, der mittleren und/oder der maximalen Intensität des zweiten Lichtspektrums betragen.

Unter einem Überlappen von Lichtspektren kann verstanden werden, dass sich die Lichtintensitätsverteilung zweier Lichtspektren überlappen. Die beiden Lichtspektren weisen also jeweils einen Wellenlängenteilbereich auf, auf den die Lichtintensität des entsprechenden Lichtspektrums teilweise verteilt ist. Jedes der Lichtspektren kann entsprechend einen Teilbereich aufweisen, der innerhalb des zugeordneten anderen Lichtspektrums liegt. Bildlich gesprochen überlagern sich Intensitätsverläufe in einem Intensitätsdiagramm, in welchem die Lichtintensität der Lichtspektren über die Wellenlängen aufgetragen ist.

Der physiologische Parameter kann einen Referenzwert umfassen, anhand dessen ein physiologischer Zustand des Objektbereichs abgeschätzt werden kann. Beispielsweise kann der Objektbereich durchblutetes Gewebe umfassen. Anhand des physiologischen Parameters kann eine Oxygenierung des Gewebes abgeschätzt werden. Der physiologische Parameter kann beispielsweise auf einem Verhältnis zweier Intensitätswerte berechnet werden, wobei jeweils ein Intensitätswert einem der beiden Lichtspektren zugeordnet werden kann. Verändert sich etwa die Oxygenierung des Gewebes, verändern sich auch die Absorptionseigenschaften des Gewebes und entsprechend auch das Lichtspektrum zurückgeworfenen Lichts. Diese Veränderung kann anhand des physiologischen Parameters abgeschätzt bzw. bestimmt werden.

Die Bilderfassungseinrichtung kann separat von der medizinischen Lichtquellenvorrichtung ausgebildet sein. Beispielsweise kann die Bilderfassungseinrichtung ein Mikroskop, ein Endoskop und/oder ein Exoskop umfassen. Das Beleuchtungslicht kann in einen Lichtleiter eingekoppelt werden und zu der Bilderfassungseinrichtung geführt werden und/oder in diese eingekoppelt werden. Die Bilderfassungseinrichtung kann über geeignete optische Elemente verfügen, sodass das Beleuchtungslicht zur Beleuchtung des Objektbereichs aus der Bilderfassungseinrichtung auskoppelbar ist. Alternativ oder zusätzlich kann das Beleuchtungslicht zur Beleuchtung des Objektbereichs verwendet werden, ohne dass es in die Bilderfassungseinrichtung eingekoppelt wird. Beispielsweise kann die Lichtquellenvorrichtung eine Beleuchtungsvorrichtung umfassen, die dazu eingerichtet ist, das Beleuchtungslicht auszukoppeln und auf den Objektbereich zu werfen. Ferner kann die Lichtquellenvorrichtung gemeinsam mit verschiedenen Bilderfassungseinrichtungen betrieben werden. Beispielsweise kann die Lichtquellenvorrichtung wahlweise mit einem Endoskop und einem Exoskop oder einem Mikroskop betrieben werden.

Die Bilderfassungseinrichtung kann eine optische Einheit umfassen, welche zumindest eine Objektivlinse umfasst. Mittels der optischen Einheit kann der Objektbereich abbildbar sein. Ferner kann die Bilderfassungseinrichtung einen Bilderfassungssensor umfassen, auf den der Objektbereich zur Bildgebung abgebildet wird.

Unter räumlicher Information kann eine Zuordnung eines Intensitätswerts zu einem Punkt des Objektbereichs verstanden werden. Es kann also eine zweidimensionale Intensitätskarte des Objektbereichs erzeugt werden. Unter spektraler Information kann eine Zuordnung dieses Intensitätswerts zu einem bestimmten Wellenlängenbereich verstanden werden. Die räumliche und spektrale Information kann dem Beleuchtungslicht bzw. dem ersten Lichtspektrum und/oder dem zweiten Lichtspektrum zugeordnet werden. Es kann also spezifisch spektrale Information erzeugt werden, die auf der Beleuchtung des Objektbereichs mit Licht mit dem ersten und/oder dem zweiten Lichtspektrum beruht.

Unter "Rückstrahlung" kann ein Strahlen jeglichen Lichts verstanden werden, das vom Objektbereich zur Bilderfassungseinrichtung gelangt und/oder das in Folge der Beleuchtung mit dem Beleuchtungslicht gestrahlt wird. Dieses Licht kann remittiertes bzw. zurückgeworfenes und emittiertes Licht bzw. Fluoreszenzlicht des Objektbereichs umfassen. Die Rückstrahlung kann also einen Teil des Beleuchtungslichts umfassen, wobei wiederum ein weiterer Teil des Beleuchtungslichts durch den Objektbereich absorbiert werden kann. Der weitere Teil kann mit einem Zustand des Objektbereichs variieren, beispielsweise mit der Oxygenierung.

Die Oxygenierung eines Gewebes im Objektbereich kann effizient und zuverlässig gemessen werden, wenn das Beleuchtungsmittel dazu eingerichtet ist, rotes Licht zu erzeugen und das Konvertierungselement dazu eingerichtet ist, rotes Licht in nahinfrarotes Licht zu wandeln. Vorteilhafterweise kann das erste Lichtspektrum in einem Wellenlängenbereich um einen Umkehrpunkt des Absorptionsspektrums von Hämoglobin liegen, oberhalb dessen die Absorption von oxygeniertem Hämoglobin oberhalb der Absorption von nicht-oxygeniertem Hämoglobin liegt. Das zweite Lichtspektrum kann oberhalb dieses Umkehrpunktes liegen. Der Umkehrpunkt kann auch als isosbestischer Punkt bezeichnet werden, bei dem die Absorption von Hämoglobin unabhängig von der Oxygenierung ist. Durch die Verwendung beispielsweise einer roten LED und eines entsprechenden Konvertierungselements können dadurch die Absorptionseigenschaften Hämoglobins gezielt ausgenutzt werden.

Unter rotem Licht kann ein Lichtspektrum im roten Wellenlängenbereich verstanden werden. Rotes Licht kann also eine Intensitätsaufteilung aufweisen, bei der die Lichtintensität zum Großteil auf den roten Wellenlängenbereich verteilt ist. Der rote Wellenlängenbereich kann Wellenlängen von etwa 600 nm bis etwa 780 nm umfassen. Nahinfrarotes Licht kann entsprechend Wellenlängen ab etwa 780 nm umfassen. Das Konvertierungselement muss jedoch nicht zwangsweise dazu eingerichtet sein, das rote Licht vollständig zu nahinfrarotem Licht zu wandeln. Gewandeltes Licht kann anteilsweise im nahinfraroten Wellenlängenbereich liegen. Anteilsweise kann gewandeltes Licht auch noch im roten Wellenlängenbereich liegen. Dieser Anteil ist vorzugsweise kleiner als der Anteil an Licht im nahinfraroten Wellenlängenbereich, insbesondere beträgt beispielsweise maximal 30 % der Lichtintensitätsverteilung, insbesondere maximal 20 %, vorzugsweise maximal 10 % oder sogar noch weniger.

Insbesondere kann sich rotes Licht von solchem Licht unterscheiden, das auch rote Spektralanteile aufweist, wie beispielsweise Weißlicht.

In anderen Worten kann das Beleuchtungsmittel dazu eingerichtet sein, Licht mit dem Ausgangslichtspektrum zu erzeugen, wobei das Ausgangslichtspektrum in einem Wellenlängenbereich zwischen 600 nm und 780 nm liegt. Das Konvertierungselement kann dazu eingerichtet sein, das Licht mit dem Ausgangslichtspektrum in Licht mit dem zweiten Lichtspektrum zu wandeln, wobei das zweite Lichtspektrum zumindest anteilig in einem Wellenlängenbereich oberhalb von 780 nm liegt. In diesem Fall kann das erste Lichtspektrum ebenfalls in einem Wellenlängenbereich zwischen 600 nm und 780 nm liegen.

Eine besonders gute Trennung der beiden Lichtspektren im Beleuchtungslicht, wodurch in weiterer Folge eine scharfe und spektral saubere Bildgebung erzielt werden kann, kann erreicht werden, wenn die medizinische Lichtquellenvorrichtung einen Bandstoppfilter zum Blockieren von Licht in einem spektralen Band, welches spektral zwischen Hauptwellenlängenbereichen des ersten Lichtspektrums und des zweiten Lichtspektrums liegt. Mittels des Bandstoppfilters kann Licht in dem Wellenlängenbereich blockiert werden, welches in dem oben beschriebenen Tal zwischen den beiden Lichtspektren liegt.

Unter einem Hauptwellenlängenbereich kann ein Wellenlängenbereich eines Lichtspektrums verstanden werden, auf den beispielsweise zumindest 80 %, insbesondere zumindest 85 %, vorzugsweise zumindest 90 %, der Lichtintensität des entsprechenden Lichtspektrums verteilt ist. Im Vergleich zu einem Lichtspektrum zugeordneten Wellenlängenbereich kann der Hauptwellenlängenbereich einen spektralen Rand oder beide spektrale Ränder des entsprechenden Wellenlängenbereichs nicht miteinschließen. In anderen Worten kann der Hauptwellenlängenbereich eines Lichtspektrums kleiner sein als der Wellenlängenbereich desselben Lichtspektrums.

Der physiologische Parameter kann besonders präzise bestimmt werden, wenn die medizinische Lichtquellenvorrichtung ein Angleichelement umfasst, welches dazu eingerichtet ist, eine Intensität des Lichts mit dem ersten Lichtspektrum und eine Intensität des Lichts mit dem zweiten Lichtspektrum anzugleichen. Durch das Angleichelement können maximale Intensitäten beider Lichtspektren einander angenähert werden, sodass in keinem der beiden entsprechenden Wellenlängenbereiche eine Überbelichtung stattfindet. Ferner kann durch das Angleichelement erreicht werden, dass Licht beispielsweise in einem roten Wellenlängenbereich und in einem nahinfraroten Wellenlängenbereich mit näherungsweise gleicher Intensität auf einen Bilderfassungssensor strahlt. Dies kann vorteilhaft sein, wenn lediglich ein Bilderfassungssensor für die Multispektralbildgebung verwendet wird.

Ein kompakter und konstruktiv einfacher Aufbau kann erreicht werden, wenn das Angleichelement durch das Konvertierungselement ausgebildet ist. Eine Transmission des Konvertierungselements kann derart gewählt sein, dass die Intensität des Lichts mit dem ersten Lichtspektrum und die Intensität des Lichts mit dem zweiten Lichtspektrum angeglichen bereitstellbar sind, insbesondere in zumindest einem Betriebszustand bereitgestellt wird. Transmittiertes Licht kann dabei Licht umfassen, das durch das Konvertierungselement nicht gewandelt wird. Die Wellenlänge transmittierten Lichts bleibt also im Wesentlichen unverändert. Das Licht mit dem ersten Lichtspektrum kann transmittiertes Licht mit dem Ausgangslichtspektrum umfassen.

Beispielsweise kann ein Intensitätsverlauf des ersten Lichtspektrums abgeflacht werden, indem das Angleichelement dazu eingerichtet ist, Licht im ersten Wellenlängenbereich bzw. im Ausgangswellenlängenbereich teilweise zu blockieren. Unter der Transmission kann entsprechend die Eigenschaft des Konvertierungselements verstanden werden, Licht, insbesondere im ersten Wellenlängenbereich und/oder im Ausgangswellenlängenbereich, durchzulassen, beziehungsweise eine Durchlässigkeit von Licht betreffen. In einigen Ausführungsformen, in denen das Konvertierungselement als Phosphorplättchen ausgebildet ist, kann die Dicke des Phosphorplättchens derart gewählt sein, dass sich die gewünschte Transmission des Konvertierungselements einstellt.

Die medizinische Lichtquellenvorrichtung kann außerdem ein weiteres Beleuchtungsmittel und ein weiteres Konvertierungselement umfassen, welche andere spektrale Eigenschaften als das Beleuchtungsmittel und das Konvertierungselement aufweisen. Dadurch kann Beleuchtungslicht bereitgestellt werden, das einen komplexeren Intensitätsverlauf aufweist. Insbesondere kann das Beleuchtungslicht vier voneinander verschiedene Hauptwellenlängenbereiche und/oder Lichtspektren aufweisen im Vergleich zu Ausführungsformen mit einem Beleuchtungsmittel und einem Konvertierungselement, bei denen das Beleuchtungslicht lediglich zwei Hauptwellenlängenbereiche und/oder Lichtspektren aufweisen kann. Der physiologische Parameter kann dadurch einerseits präziser bestimmt werden. Andererseits könnten auch zwei oder mehr physiologische Parameter simultan bestimmt werden.

Zudem kann das Konvertierungselement dazu eingerichtet sein, zeitverzögert das Licht mit dem Ausgangslichtspektrum in das Licht mit dem zweiten Lichtspektrum zu wandeln. Durch eine geeignete Ansteuerung des Bildsensors einer Bildgebungsvorrichtung kann dann nacheinander ein Bild nach Beleuchtung mit Licht mit dem ersten Lichtspektrum und mit Licht mit dem zweiten Lichtspektrum erzeugt werden. Durch ein alternierendes Ein- und Ausschalten des Beleuchtungsmittels kann das Lichtspektrum des Beleuchtungslichts ebenfalls alternierend sein. Dann können, wie bereits beschrieben, zwei aufeinanderfolgende unabhängige Bilder mittels eines einzigen Bildsensors aufgenommen werden und die für das erste und das zweite Lichtspektrum spezifischen Bilder erfasst werden. Der Bildsensor kann insbesondere eine getrennte Belichtungsregelung, eine Anpassung an Rolling-Shutter Effekte und/oder dergleichen aufweisen. Das Konvertierungselement kann etwa dazu eingerichtet sein, das Licht beruhend auf Fluoreszenz zu wandeln. Ferner kann es dazu eingerichtet sein, mit einer Zeitverzögerung von zumindest 10 ms das Licht mit dem ersten Lichtspektrum in das Licht mit dem zweiten Lichtspektrum zu wandeln.

Alternativ kann das Konvertierungselement dazu eingerichtet sein, das Licht mit dem ersten Lichtspektrum in das Licht mit dem zweiten Lichtspektrum im Wesentlichen in Echtzeit zu wandeln. Unter "Echtzeit" kann eine Zeitverzögerung von maximal 100 µs verstanden werden.

Zudem können die Bilddaten lichtspektrumsspezifisch für das erste Lichtspektrum und das zweite Lichtspektrum erzeugbar sein. Es kann also eine klare spektrale Trennung der Bilddaten für das erste und das zweite Lichtspektrum vorgesehen sein. Dadurch kann der physiologische Parameter genau bestimmt werden, da ein Einfluss von Licht außerhalb der entsprechenden Lichtspektren auf eine gemessene Intensität minimiert werden kann. Die gemessene Intensität kann folglich genau dem ersten bzw. dem zweiten Lichtspektrum zugeordnet werden. In anderen Worten kann jeweils für das erste Lichtspektrum und das zweite Lichtspektrum ein Bild erzeugbar sein, wobei das entsprechende Bild insbesondere auf zurückgeworfenem Licht des Objektbereichs in dem entsprechendem Wellenlängenbereich beruht.

In einigen Ausführungsformen umfasst die Bilderfassungseinrichtung zwei Bilderfassungssensoren, welche in unterschiedlichen Wellenlängenbereichen lichtempfindlich sind. Mittels der zwei Bildsensoren können Bilddaten lichtspektrumsspezifisch für das erste Lichtspektrum und das zweite Lichtspektrum erzeugbar sein. Jeder der Bilderfassungssensoren kann etwa dazu eingerichtet sein, Bilddaten entweder für das erste Lichtspektrum oder das zweite Lichtspektrum zu erfassen. Die Bilderfassungseinrichtung kann etwa eine Eingangsoptik für beide Bilderfassungssensoren gemeinsam aufweisen. Das mittels der Eingangsoptik in die Bilderfassungseinrichtung eingekoppelte Licht kann mittels eines dichroitischen Elements der Bilderfassungseinrichtung aufteilbar sein und jeweils ein Teil auf einen ersten Bilderfassungssensor und einen zweiten Bilderfassungssensor führbar sein.

Eine kompakte und konstruktiv einfache Bildererfassungseinrichtung kann bereitgestellt werden, wenn sie einen Bilderfassungssensor umfasst, der dazu eingerichtet ist, die Bilddaten für das erste Lichtspektrum und das zweite Lichtspektrum zu erfassen. Der eine Bilderfassungssensor kann also dazu eingerichtet sein, die spektral voneinander getrennten Bilddaten zu erfassen. Wird der Objektbereich etwa mittels roten und nahinfraroten Lichts beleuchtet, kann der eine Bilderfassungssensor dazu eingerichtet sein, ein Bild spezifisch für das rote Licht und ein Bild spezifisch für das nahinfrarote Licht zu erfassen. Die Bilddaten können mittels des Bilderfassungssensors, insbesondere lichtspektrumsspezifisch, gemeinsam erfassbar sein.

In einigen Ausführungsformen umfasst der Bilderfassungssensor einen Farbfilterarray, welcher Segmente umfasst, welche in unterschiedlichen Wellenlängenbereichen des sichtbaren Lichts unterschiedlich lichtdurchlässig sind, wobei die Segmente zumindest in einem nahinfraroten Wellenlängenbereich zumindest annähernd gleich lichtdurchlässig sind. Solche Farbfilterarrays sind häufig in herkömmlichen Bilderfassungseinrichtungen verbaut. Es kann also die Multispektralbildgebung unter Ausnutzung spektraler Eigenschaften bekannter Farbfilterarrays und Bilderfassungssensoren durchgeführt werden. Das Farbfilterarray kann etwa eine Bayer-Matrix umfassen. Dabei haben die Erfinder erkannt, dass sich die spektralen Eigenschaften der Bayer-Matrix und der Bilderfassungssensors gezielt für die Oxygenierungsmessung ausnutzen lassen.

Ferner kann die Bilderfassungseinrichtung einen optischen Filter umfassen, der dazu eingerichtet ist, Licht mit dem ersten Lichtspektrum zu blockieren. Beispielsweise kann dies bei einer Fluoreszenzbildgebung ausgenutzt werden. Remittiertes Licht im ersten Wellenlängenbereich kann entsprechend blockiert werden, sodass es nicht auf den Bilderfassungssensor trifft. Es kann dann etwa ein Fluoreszenzfarbstoff im Objektbereich vorgesehen sein, der Licht im Wellenlängenbereich des ersten Lichtspektrums absorbiert und wandelt. Ebenso denkbar ist, dass eine Autofluoreszenz des Gewebes gemessen wird. Der optische Filter kann beispielsweise vor der Eingangsoptik der Bilderfassungseinrichtung oder innerhalb der Bilderfassungseinrichtung vor dem Bilderfassungssensor und hinter der Eingangsoptik angeordnet sein.

Außerdem kann das medizinisches Bildgebungssystem eine Auswerteeinheit umfassen, die dazu eingerichtet ist, nach Maßgabe der räumlichen und spektralen Informationen den physiologischen Parameter zu bestimmen. Vorteilhafterweise können dadurch mittels der Auswerteeinheit spektrale und/oder optische Eigenschaften des Objektbereichs analysiert werden.

Zudem kann die Auswerteeinheit dazu eingerichtet sein, zumindest zwei unterschiedliche Intensitätswerte miteinander zu vergleichen, die sich auf das erste Lichtspektrum und das zweite Lichtspektrum beziehen, um den physiologischen Parameter zu bestimmen. In einfacher und effizienter Weise kann dadurch die Oxygenierung bestimmt werden. Durch das Bilden eines Verhältnisses der unterschiedlichen Intensitätswerte kann eine Genauigkeit und Empfindlichkeit in der Oxygenierungsbestimmung verbessert werden.

Eine zuverlässige und sensible Oxygenierungsbestimmung kann erreicht werden, wenn die Auswerteeinheit dazu eingerichtet ist, den physiologischen Parameter anhand wenigstens zweier unterschiedlicher spektraler Stützstellen zu bestimmen, wobei eine erste spektrale Stützstelle im Bereich eines ersten Intensitätsmaximums des ersten Lichtspektrums und eine zweite spektrale Stützstelle im Bereich eines zweiten Intensitätsmaximums des zweiten Lichtspektrums liegt. Eine spektrale Stützstelle kann einen Intensitätsverlauf betreffen. Es kann also etwa im roten Wellenlängenbereich und/oder im nahinfraroten Wellenlängenbereich ein über einen Wellenlängenbereich integrierter Intensitätswert zur Oxygenierungsbestimmung verwendet werden. Die spektrale Stützstelle kann ebenso einen Intensitätswert bei genau einer Wellenlänge und/oder in einem schmalen Wellenlängenbereich betreffen. "Schmal" kann in dem Zusammenhang eine Bandbreite von maximal 10 nm, insbesondere 5 nm, bevorzugt 2 nm, bedeuten. Bei der Oxygenierungsbestimmung kann dadurch ausgenutzt werden, dass die Absorption oxygenierten und nicht-oxygenierten Hämoglobins oberhalb einer Wellenlänge von 800 nm bis etwa 920 nm relativ konstant ist. Über diesen Wellenlängenbereich kann die Intensität integriert werden, um eine hochqualitative Messung und Parameterbestimmung durchzuführen.

In einigen Ausführungsformen umfasst das medizinische Bildgebungssystem, insbesondere die Bilderfassungseinrichtung, einen Endoskopschaft mit einem proximalen Abschnitt und einem distalen Abschnitt, wobei die Lichtquellenvorrichtung im distalen Abschnitt angeordnet ist. Dadurch, dass die Lichtquellenvorrichtung das Konvertierungselement umfasst, kann ein kompaktes, kleinkalibriges Endoskop bereitgestellt werden, mittels dessen ein physiologischer Parameter bestimmt werden kann. Die Lichtquellenvorrichtung kann in dem Fall etwa eine LED umfassen, die an einer distalen Endfläche des Endoskopschafts angeordnet ist. In Kombination mit nur einem Bilderfassungssensor und dem Farbfilterarray kann dann in bereits dargelegter Weise der physiologische Parameter bestimmt werden. Folglich kann ein kompaktes Endoskop bereitgestellt werden, mittels dessen Multispektralbildgebung durchgeführt werden kann und insbesondere die Oxygenierung von einem Gewebe im Objektbereich bestimmt werden kann. Hierzu müssen lediglich ein Beleuchtungsmittel und ein Bilderfassungssensor vorgesehen sein.

Im Folgenden wird die vorliegende Erfindung anhand der beigefügten Figuren beispielhaft beschrieben. Die Zeichnung, die Beschreibung und die Ansprüche enthalten zahlreiche Merkmale in Kombination. Der Fachmann wird die Merkmale zweckmäßigerweise auch einzeln betrachten und im Rahmen der Ansprüche sinnvoll in Kombination verwenden.

Falls von einem bestimmten Objekt mehr als ein Exemplar vorhanden ist, ist ggf. nur eines davon in den Figuren und in der Beschreibung mit einem Bezugszeichen versehen. Die Beschreibung dieses Exemplars kann entsprechend auf die anderen Exemplare von dem Objekt übertragen werden. Sind Objekte insbesondere mittels Zahlenwörtern, wie beispielsweise erstes, zweites, drittes Objekt etc. benannt, dienen diese der Benennung und/oder Zuordnung von Objekten. Demnach können beispielsweise ein erstes Objekt und ein drittes Objekt, jedoch kein zweites Objekt umfasst sein. Allerdings könnten anhand von Zahlenwörtern zusätzlich auch eine Anzahl und/oder eine Reihenfolge von Objekten ableitbar sein.

Es zeigen:
- Fig. 1: eine schematische Darstellung eines Bildgebungssystems umfassend eine medizinische Lichtquellenvorrichtung und eine Bilderfassungseinrichtung;
- Fig. 2: eine schematische Darstellung der medizinischen Lichtquellenvorrichtung;
- Fig. 3: eine schematische Darstellung eines Diagramms, in dem ein Intensitätsverlauf von Licht mit einem ersten Lichtspektrum und einem zweiten Lichtspektrum aufgetragen ist;
- Fig. 4: eine schematische Darstellung eines Diagramms, in dem ein Absorptionsverhalten oxygenierten und nicht-oxygenierten Hämoglobins aufgetragen ist;
- Fig. 5: eine schematische Darstellung eines Diagramms, in dem ein Intensitätsverlauf von Licht mit einem ersten Lichtspektrum und einem zweiten Lichtspektrum aufgetragen ist, wobei das Licht zudem durch einen Bandstoppfilter durchgetreten ist;
- Fig. 6: eine schematische Darstellung einer weiteren Ausführungsform eines Bildgebungssystems umfassend eine medizinische Lichtquellenvorrichtung und eine Bilderfassungseinrichtung
- Fig. 7: eine schematische Darstellung eines Farbfilterarrays; und
- Fig. 8: ein schematisches Ablaufdiagramm eines Verfahrens zum Betrieb einer medizinischen Lichtquellenvorrichtung und/oder eines medizinischen Bildgebungssystems.

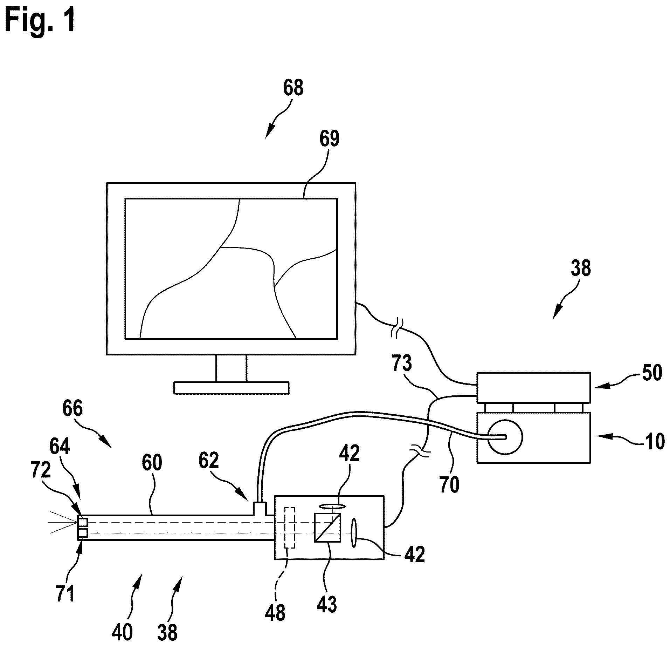

Fig. 1 zeigt eine schematische Darstellung eines medizinischen Bildgebungssystems 38. Das Bildgebungssystem 38 umfasst eine Bilderfassungseinrichtung 40, eine medizinische Lichtquellenvorrichtung 10, eine Auswerteeinheit 50 und eine Anzeigeeinrichtung 68. Die Bilderfassungseinrichtung 40 ist als Endoskop 66 ausgebildet und umfasst einen Endoskopschaft 60, welcher einen distalen Abschnitt 64 und einen proximalen Abschnitt 62 aufweist. Der Endoskopschaft 60 ist dazu eingerichtet, für eine Bildgebung teilweise in eine Kavität eines Körpers eines Patienten eingeführt zu werden. Genauer wird der distale Abschnitt 64 in die Kavität eingeführt.

An dem distalen Abschnitt 64 ist eine Eingangsoptik 71 und eine Beleuchtungsvorrichtung 72 angeordnet. Mittels der Beleuchtungsvorrichtung 72 kann ein Objektbereich mit Beleuchtungslicht beleuchtet werden. Das entsprechende Beleuchtungslicht wird mittels der medizinischen Lichtquellenvorrichtung 10 bereitgestellt, mittels eines Lichtleiters 70 zu der Bilderfassungseinrichtung 40 geführt und in diese eingekoppelt. Innerhalb der Bilderfassungseinrichtung 40 wird das Beleuchtungslicht beispielsweise mittels eines Lichtleiters oder eines optischen Systems (beide nicht dargestellt) zu der Beleuchtungsvorrichtung 72 geführt. In Folge der Beleuchtung mit dem Beleuchtungslicht wirft ein Objektbereich (nicht dargestellt) Licht zu der Bilderfassungseinrichtung 40 zurück, welches teilweise mittels der Eingangsoptik 71 in den Endoskopschaft 60 eingekoppelt wird. Durch die Eingangsoptik 71 wird der Objektbereich zudem abgebildet. Das so gesammelte Licht wird entlang des Endoskopschafts 60 geführt und auf einen Strahlteiler 43 geleitet. Dieser ist dazu eingerichtet, eintreffendes Licht in zwei spektrale Bestandteile zu teilen. Jeweils einer der beiden spektralen Bestandteile wird auf jeweils einen Bilderfassungssensor 42 geführt und der Objektbereich entsprechend auf die Bilderfassungssensoren 42 abgebildet. Das jeweils auf die unterschiedlichen Bilderfassungssensoren 42 fallende Licht weist jedoch unterschiedliche Lichtspektren auf bzw. umfasst Licht in unterschiedlichen Wellenlängenbereichen. Dadurch kann mittels der Bilderfassungseinrichtung 40 multispektrale Bildgebung durchgeführt werden.

Entsprechend werden mit den Bilderfassungssensoren 42 Bilddaten erzeugt, die räumliche und spektrale Informationen umfassen. Diese Bilddaten werden mittels eines Kabels 73 an die Auswerteeinheit 50 geleitet. Die Auswerteeinheit 50 ist dazu eingerichtet, anhand der Bilddaten einen physiologischen Parameter zu bestimmen. Dies wird im Folgenden noch genauer beschrieben.

Die Auswerteeinheit 50 ist zudem mit der Anzeigeeinrichtung 68 verbunden, die dazu eingerichtet ist, eine Darstellung 69 des Objektbereichs zu erzeugen. Ein Benutzer kann über die Anzeigeeinrichtung 68 beispielsweise ein Gewebe einsehen, das sich innerhalb der Kavität des Patienten und in dem abgebildeten Objektbereich befindet. Ferner kann die Darstellung 69 eine Falschfarbendarstellung umfassen. Wird etwa basierend auf der oben beschriebenen Multispektralbildgebung der physiologische Parameter bestimmt, kann dieser räumlich aufgelöst in der Falschfarbendarstellung wiedergegeben werden. Betrifft der physiologische Parameter etwa eine Oxygenierung des Gewebes, so kann ein Oxygenierungsgrad in der Falschfarbendarstellung dargestellt werden und der Benutzer die Oxygenierung an der Anzeigevorrichtung 68 nahezu in Echtzeit einsehen. Denkbar ist auch, dass der physiologische Parameter Gewebeveränderungen, beispielsweise einen Tumor, betrifft. Ebenso sind weitere Anwendungsfälle denkbar.

Zudem kann ein optischer Filter 48 vor dem Strahlteiler 43 angeordnet sein. Denkbar ist auch, dass dieser bedarfsweise vor dem Strahlteiler 43 anordenbar ist. Der optische Filter 48 könnte dann durch den Benutzer in das Endoskop 66 eingeschoben werden. Der optische Filter 48 ist im vorliegenden Beispiel ein optischer Hochpassfilter, der dazu eingerichtet ist, Licht oberhalb einer Grenzfrequenz durchzulassen und unterhalb der Grenzfrequenz zu blockieren. Die Grenzfrequenz wird in Abhängigkeit von spektralen Eigenschaften des Beleuchtungslichts gewählt. Der optische Filter 48 kann entsprechend ein Beobachtungsfilter für Fluoreszenzbildgebung sein. Dieser kann auch als Bandstoppfilter ausgebildet sein, ähnlich dem Bandstoppfilter 22. Wird etwa Fluoreszenzbildgebung durchgeführt, wird die Grenzfrequenz näherungsweise so gewählt, dass sie oberhalb einer Absorptionswellenlänge eines für die Bildgebung verwendeten Fluorophors liegt. Remittiertes Licht des Objektbereichs wird also mittels des optischen Filters 48 blockiert, emittiertes Licht wird jedoch durchgelassen.

Die Lichtquellenvorrichtung 10 und die Auswerteeinheit 50 sind als eigenständig bewegbare, modulartige Einheiten ausgebildet, die bedarfsweise in einem Operationssaal vorsehbar sind. Die Auswerteeinheit 50 bildet zudem eine Steuereinheit für das Endoskop 66 aus, mittels derer Bildaufnahmefunktionen und/oder dergleichen gesteuert werden.

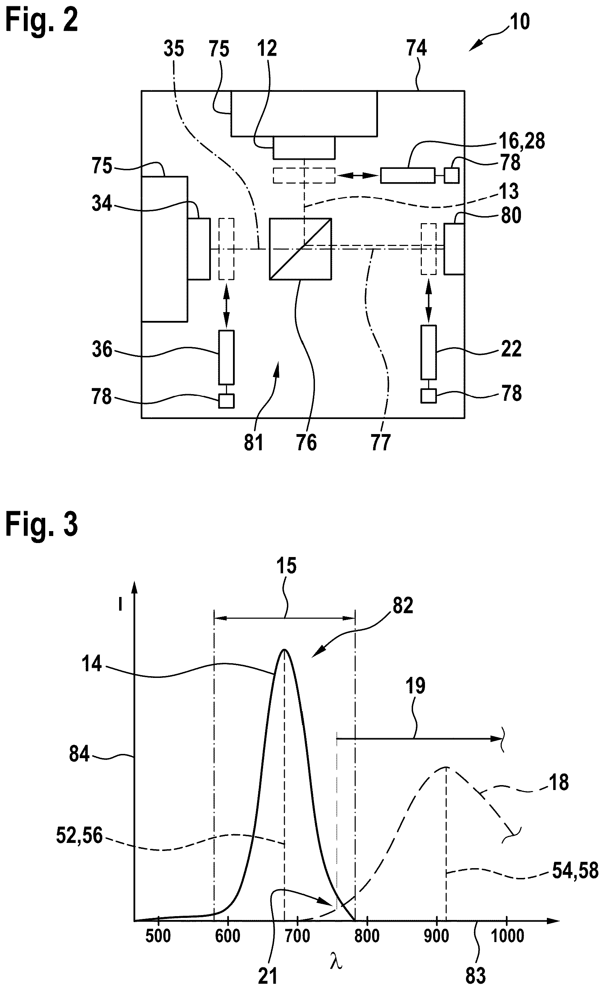

Fig. 2 zeigt eine schematische Darstellung der medizinischen Lichtquellenvorrichtung 10, die dazu eingerichtet ist, das Beleuchtungslicht bereitzustellen. Die Lichtquellenvorrichtung 10 weist ein Gehäuse 74 auf, das die weiteren Elemente der Lichtquellenvorrichtung 10 einhaust. Sie umfasst ein Beleuchtungsmittel 12 und ein weiteres Beleuchtungsmittel 34. Diese sind auf jeweils einer Kühlvorrichtung 75 in Form eines Kühlkörpers angeordnet. Die Beleuchtungsmittel 12, 34 sind jeweils dazu eingerichtet, in einen Innenraum 81 der Lichtquellenvorrichtung 10 Licht entlang einer optischen Achse 13, 35 zu emittieren. An einer Schnittstelle der optischen Achsen 13, 35 beider Beleuchtungsmittel 12, 34 ist ein Strahlkombinierer 76 der Lichtquellenvorrichtung 10 angeordnet, der dazu eingerichtet ist, das erzeugte Licht der Beleuchtungsmittel 12, 34 auf einen gemeinsamen optischen Pfad 77 zu führen und zu einer optischen Schnittstelle 80 zu leiten. Das so kombinierte Licht ist dann als Beleuchtungslicht bereitstellbar. An der optischen Schnittstelle 80 wird der Lichtleiter 70 angeschlossen, um das Beleuchtungslicht abzugreifen und zu dem Endoskop 66 oder einer anderen, nicht dargestellten, Bilderfassungseinrichtung zuzuführen. Denkbar ist beispielsweise, dass mittels der Lichtquellenvorrichtung 10 ebenso ein Mikroskop und/oder ein Exoskop betrieben werden kann.

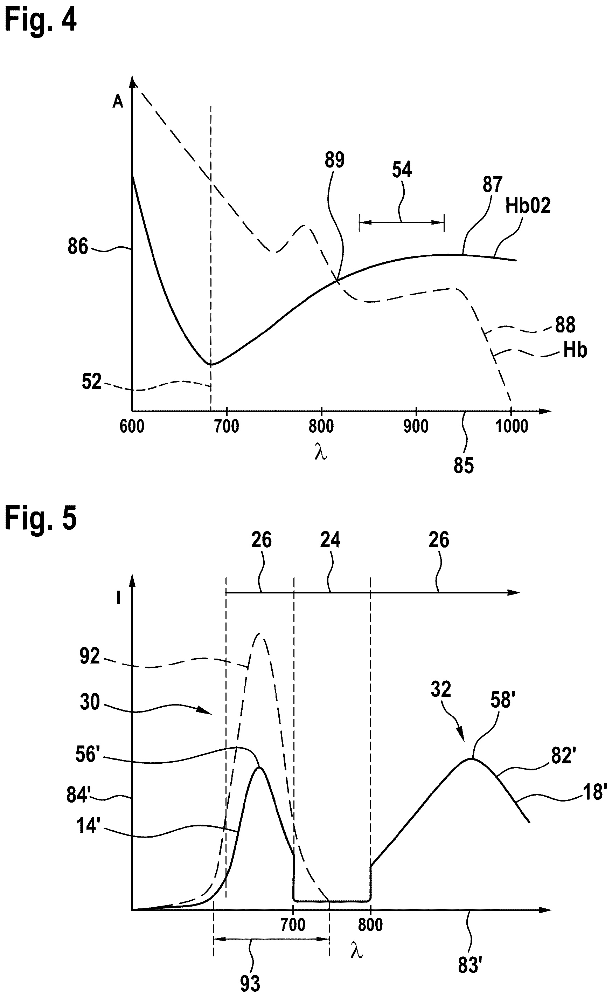

Vor den Beleuchtungsmitteln 12, 34 ist zudem jeweils ein Konvertierungselement 16, 36 bedarfsweise anordenbar. Das Konvertierungselement 16, 36 ist dazu eingerichtet, das von dem zugeordneten Beleuchtungsmittel 12, 34 bereitgestellte Licht teilweise spektral zu wandeln. Dies wird im Folgenden noch genauer beschrieben. Soll das entsprechende Konvertierungselement 16, 36 verwendet werden, kann es mittels einer zugeordneten Einschiebevorrichtung 78 in die optische Achse 13, 35 des jeweiligen Beleuchtungsmittels 12, 34 geschoben werden. In einigen nicht dargestellten Ausführungsformen ist das Konvertierungselement 16, 36 unbeweglich vor dem entsprechenden Beleuchtungsmittel 12, 34 angeordnet. Das Konvertierungselement 16, 36 ist beispielsweise als Phosphorplättchen ausgebildet. Ferner ist zudem ebenfalls mittels einer Einschiebevorrichtung 78 ein Bandstoppfilter 22 in den gemeinsamen optischen Pfad 77 einschiebbar. Eine Funktion des Bandstoppfilters 22 ist im Zusammenhang mit der Fig. 5 beschrieben. Denkbar ist auch, dass jeweils ein Bandstoppfilter gemeinsam mit dem entsprechenden Konvertierungselement 16, 36 in die zugeordnete optische Achse 13, 35 geschoben wird (nicht dargestellt). Der entsprechende Bandstoppfilter 22 weist ein Stoppband auf, das spektral hinsichtlich der Lichtspektren des zugeordneten Beleuchtungsmittels 12, 34 und Konvertierungselements 16, 36 gewählt ist.

Das Konvertierungselement 16, 36 weist jeweils eine Transmission auf, was bedeutet, dass es einen Anteil des auftreffenden Lichts zumindest im Wesentlichen ohne eine spektrale Verschiebung durchlässt. In diesem Zusammenhang wird auf die Fig. 3 bis 5 verwiesen.

Im Zusammenhang mit den Fig. 3 und 4 ist die Funktionsweise des Beleuchtungsmittels 12 und des Konvertierungselements 16 beschrieben. Das weitere Beleuchtungsmittel 34 und Konvertierungselement 36 weist eine entsprechende Funktionsweise in einem anderen Wellenlängenbereich auf. In anderen Worten weisen diese beiden Elemente 34, 36 andere spektrale Eigenschaften auf als das Beleuchtungsmittel 12 und das Konvertierungselement 16.

Es ist ebenso denkbar, dass die Lichtquellenvorrichtung 10 anstelle des weiteren Beleuchtungsmittels 34 und Konvertierungselements 36 eine Weißlicht-LED aufweist. Diese kann dann für eine Weißlichtbildgebung verwendet werden, wobei das Beleuchtungsmittel 12 und das Konvertierungselement 16 für eine Multispektralbildgebung gemäß den folgenden Ausführungen verwendet wird.

Das Beleuchtungsmittel 12 und das Konvertierungselement 16 sind gemäß den folgenden Ausführungen spezifisch für die Oxygenierungsbestimmung eines Gewebes im Objektbereich ausgebildet. Fig. 3 zeigt ein schematisches Diagramm mit einem Intensitätsverlauf 82 (bzw. eines Gesamtlichtspektrums) des Beleuchtungslichts, das die Lichtquellenvorrichtung 10 bereitstellt. Mittels dieses Beleuchtungslichts wird der Objektbereich beleuchtet. Fig. 4 zeigt ein schematisches Diagramm mit Kurven 87, 88, die eine Absorption oxygenierten Hämoglobins (Hb02) und nicht-oxygenierten Hämoglobins (Hb) illustrieren.

Zunächst bezugnehmend auf die Fig. 4 ist ein Diagramm zu sehen, das eine Abszisse 85, an der die Wellenlänge aufgetragen ist, und eine Ordinate 86, an der die Absorption aufgetragen ist, aufweist. Man erkennt, dass bis zu einem Umkehrpunkt 89, der bei einer Wellenlänge von etwa 790 nm liegt, nicht-oxygeniertes Hämoglobin stärker absorbiert, wobei oberhalb des Umkehrpunkts 89 oxygeniertes Blut stärker absorbiert. Zudem erkennt man ein Minimum in der Absorption oxygenierten Hämoglobins bei einer Wellenlänge von etwa 660 nm. Ferner ist in einem Bereich ab einer Wellenlänge von 800 nm bis zu einer Wellenlänge von etwa 900 nm die Absorption oxygenierten und nicht-oxygenierten Hämoglobins in etwa konstant. Diese spektralen Eigenschaften werden gezielt ausgenutzt, um die Oxygenierung des Gewebes im Objektbereich zu bestimmen. Beleuchtungslicht, das auf durchblutetes Gewebe geworfen wird, wird entsprechend abhängig von der Oxygenierung des Hämoglobins teilweise absorbiert und zurückgeworfen. Das zurückgeworfene Licht weist also einen von der Oxygenierung des Gewebes abhängigen Intensitätsverlauf auf, der zur Bestimmung des physiologischen Parameters verwendet werden kann.

In dem in Fig. 3 dargestellten Fall ist als Beleuchtungsmittel 12 eine rote LED vorgesehen, die einen Emissionspeak (nicht dargestellt) mit einem Intensitätsmaximum (nicht dargestellt) bei einer Wellenlänge von etwa 660 nm aufweist. Um dieses Intensitätsmaximum ist die Intensität in einem Ausgangswellenlängenbereich von 580 nm bis 780 nm annähernd normalverteilt. Licht mit einem derartigen Ausgangslichtspektrum wird durch das Konvertierungselement zumindest teilweise in Licht mit dem zweiten Lichtspektrum 18 gewandelt. Ein Anteil des Lichts wird jedoch nicht gewandelt und weist ein erstes Lichtspektrum 14 auf. Um Licht mit dem ersten Lichtspektrum 14 für das Beleuchtungslicht zu verwenden, ist das Konvertierungselement 16, wie bereits beschrieben, teildurchlässig ausgebildet. Es ist also dazu eingerichtet, Licht mit dem Ausgangslichtspektrum teilweise abgeschwächt durchzulassen. Das durchgelassene Licht weist entsprechend das erste Lichtspektrum 14 auf. Dieses Licht entspricht spektral dem Ausgangslicht, es liegt also im gleichen Wellenlängenbereich und ist nicht spektral verschoben; im Gegensatz zum Licht mit dem zweiten Lichtspektrum, das mittels des Konvertierungselements 16 gewandelt wird und spektral zu größeren Wellenlängen verschoben wird.

In dem Diagramm der Fig. 3 sind ein entsprechendes erstes Lichtspektrum 14 und zweites Lichtspektrum 18 schematisch dargestellt. Das Diagramm umfasst eine Abszisse 83, an der Wellenlängen aufgetragen sind, und eine Ordinate 84, an der eine Lichtintensität aufgetragen ist. Man erkennt das erste Lichtspektrum 14 von Licht, das durch die rote LED bzw. das Beleuchtungsmittel 12 erzeugt wird und durch das Konvertierungselement 16 abgeschwächt durchgelassen wird. Das erste Lichtspektrum 14 weist einen ersten Wellenlängenbereich 15 von etwa 580 nm bis 780 nm auf, der auch dem Ausgangswellenlängenbereich entspricht. Zudem weist das erste Lichtspektrum 14 entsprechend ein erstes Intensitätsmaximum 56 bei einer Wellenlänge von etwa 660 nm auf. Ferner erkennt man ein zweites Lichtspektrum 18 mit einem entsprechendem zweiten Wellenlängenbereich 19. Dieser Wellenlängenbereich 19 beginnt bei einer Wellenlänge von etwa 740 nm und erstreckt sich über den dargestellten Bereich hinaus. Der zweite Wellenlängenbereich 19 beginnt also im Wellenlängenbereich roten Lichts und erstreckt sich bis in den Wellenlängenbereich infraroten, insbesondere nahinfraroten Lichts, hinein. Das zweite Lichtspektrum 18 weist ein zweites Intensitätsmaximum 58 bei einer Wellenlänge von etwa 910 nm auf. Beide Lichtspektren 14, 18 gemeinsam ergeben das Gesamtlichtspektrum des Beleuchtungslichts.

Licht mit dem zweiten Lichtspektrum 18 wird durch das Konvertierungselement 16 erzeugt, indem dieses dazu eingerichtet ist, Licht mit dem Ausgangslichtspektrum teilweise in Licht mit einem zweiten Lichtspektrum 18 zu wandeln. Das Konvertierungselement ist also dazu eingerichtet, rotes Licht in nahinfrarotes Licht zu wandeln. In der Fig. 3 erkennt man, dass das erste Lichtspektrum 14 in einem kurzwelligeren Wellenlängenbereich als das zweite Lichtspektrum 18 liegt, das erste Lichtspektrum 14 das zweite Lichtspektrum 18 höchstens zu 50 % überlappt, genauer zu etwa 1 % im dargestellten Fall, und das erste Lichtspektrum 14 im Vergleich zu dem zweiten Lichtspektrum 18 eine geringere Bandbreite 20 aufweist.

In bereits beschriebener Weise wird mittels der Bilderfassungseinrichtung 40 multispektrale Bildgebung in Folge der Beleuchtung des Objektbereichs mittels Beleuchtungslichts umfassend Licht mit dem ersten und dem zweiten Lichtspektrum 14, 18 durchgeführt. Mittels der Bilderfassungseinrichtung 40 wird dabei der Objektbereich abgebildet und es werden Bilddaten des Objektbereichs erzeugt. Die Bilddaten umfassen räumliche und spektrale Informationen, welche sich auf eine Rückstrahlung des Objektbereichs in Folge der Beleuchtung mit Licht mit dem ersten Lichtspektrum 14 und dem zweiten Lichtspektrum 18 beziehen. Die Rückstrahlung ist abhängig von der Oxygenierung des Gewebes im Objektbereich. Es werden also Bilddaten lichtspektrumsspezifisch für das erste und das zweite Lichtspektrum 14, 18 erzeugt. Dies geschieht in diesem Ausführungsbeispiel mittels zweier Bilderfassungssensoren 42, die in unterschiedlichen Wellenlängenbereichen, genauer dem ersten Wellenlängenbereich 15 und dem zweiten Wellenlängenbereich 19, lichtempfindlich sind (siehe Fig. 1). Es werden also zwei Bilder erzeugt, die auf Licht in unterschiedlichen Wellenlängenbereichen beruhen.

Mittels der Auswerteeinheit 50 werden die beiden Bilder ausgewertet, um den physiologischen Parameter zu bestimmen. Bei der Bestimmung des physiologischen Parameters wird die wellenlängenabhängige Absorption Hämoglobins ausgenutzt. Zur Bestimmung des physiologischen Parameters, in diesem Fall betreffend die Oxygenierung von Gewebe im Objektbereich, werden mittels der Auswerteeinheit 50 zwei unterschiedliche Intensitätswerte miteinander verglichen. Diese beziehen sich auf das erste Lichtspektrum 14 und das zweite Lichtspektrum 18 und werden aus den entsprechenden lichtspektrumsspezifischen Bilddaten ausgelesen.

Genauer wird der physiologische Parameter anhand zweier unterschiedlicher spektraler Stützstellen 52, 54 bestimmt (siehe Fig. 3), wobei eine erste spektrale Stützstelle 52 im Bereich des ersten Intensitätsmaximums 56 des ersten Lichtspektrums 14 und eine zweite spektrale Stützstelle 54 im Bereich eines zweiten Intensitätsmaximums 58 des zweiten Lichtspektrums 18 liegt. Da das oxygenierte Hämoglobin bei etwa 660 nm ein Absorptionsminimum aufweist, wird die Lichtintensität bei dieser Wellenlänge als spektrale Stützstelle 52 verwendet. Wie bereits beschrieben weisen oxygeniertes und nicht-oxygeniertes Hämoglobin oberhalb von 800 nm eine näherungsweise konstante Absorption auf. Daher wird bei der zweiten spektralen Stützstelle 54 die Intensität über den Wellenlängenbereich integriert, in dem die Absorption näherungsweise konstant ist. Für die beiden spektralen Stützstellen 52, 54 werden, da die Bilddaten lichtspektrumsspezifisch ausgelesen werden, die Bilddaten spektral getrennt voneinander erzeugt. In anderen Worten wird jeweils ein eigenes Bild mit räumlicher und spektraler Information in den Wellenlängenbereichen der beiden spektralen Stützstellen 52, 54 erzeugt.

Zur Parameterbestimmung werden entsprechend bestimmte Intensitätswerte beider spektraler Stützstellen 52, 54 miteinander in ein Verhältnis gesetzt, wodurch eine relative Oxygenierung Hämoglobins bestimmt wird. Diese wird ortsaufgelöst über die Bildpunkte der Bilder des Objektbereichs bestimmt. Das heißt, für jeden Bildpunkt wird ein Parameter bestimmt, der dann in der Falschfarbendarstellung dargestellt wird.

Die Fig. 5 zeigt ein schematisches Diagramm mit einem Intensitätsverlauf 82' von Beleuchtungslicht, wobei der Bandstoppfilter 22 in dem optischen Pfad 77 angeordnet ist. Zudem ist ein Angleichelement 28 vorgesehen, das integral mit dem Konvertierungselement 16 ausgebildet ist (siehe Fig. 2). Der Intensitätsverlauf 82' entspricht im Wesentlichen dem Intensitätsverlauf 82. Daher wird überwiegend auf Unterschiede eingegangen. Das schematische Diagramm umfasst eine Abszisse 83', an der Wellenlängen aufgetragen sind, und eine Ordinate 84', an der eine Lichtintensität aufgetragen ist.

Zusätzlich erkennt man in dem Diagramm das Ausgangslichtspektrum 92 mit seinem Ausgangswellenlängenbereich 93. Dieses entspricht dem bereits beschriebenen Ausgangslichtspektrum.

Im Ausgangswellenlängenbereich 93 ist das erste Lichtspektrum 14 zu sehen, das ein abgeschwächtes Ausgangslichtspektrum 92 ist. Dieses wird durch den Durchtritt durch das Angleichelement 28 abgeschwächt, welches durch das Konvertierungselement 16 ausgebildet ist. Genauer ist das Konvertierungselement 16 als Phosphorplättchen ausgebildet, das eine bestimmte Dicke aufweist. Durch eine Wahl einer Dicke des Phosphorplättchens kann eine Transmission eingestellt werden, welche bestimmt, wie stark das Licht mit dem Ausgangslichtspektrum 92 abgeschwächt wird.

Im vorliegenden Fall ist die Dicke des Phosphorplättchens so gewählt, dass eine Intensität 30 des Lichts mit dem ersten Lichtspektrum 14' und eine Intensität 32 des Lichts mit einem zweiten Lichtspektrum 18' angeglichen wird. Genauer werden Intensitätsmaxima 56', 58' des ersten und des zweiten Lichtspektrums 14', 18' derart angepasst, dass sie in etwa gleich hoch sind. Je nach Anwendung sind auch andere Verhältnisse der Intensitäten 30, 32 oder der Intensitätsmaxima 56', 58' denkbar. Diese können, wie bereits beschrieben, durch eine geeignete Wahl der Transmission des Konvertierungselements 16 gezielt eingestellt werden.

Der Bandstoppfilter 22 blockiert Licht in einem spektralen Band 24, das von einer Wellenlänge von 700 nm bis zu einer Wellenlänge von 800 nm reicht. Dieses spektrale Band 24 ist spektral zwischen den Intensitätsmaxima 56', 58' der beiden Lichtspektren 14', 18' angeordnet. Genauer ist das spektrale Band 24 zwischen Hauptwellenlängenbereichen 26 des ersten Lichtspektrums 14' und des zweiten Lichtspektrums 18' angeordnet. Die Hauptwellenlängenbereiche 26 umfassen jeweils zumindest 85 % der integrierten Intensität im dem entsprechenden Lichtspektrum 14', 18' zugeordneten Wellenlängenbereich (siehe Fig. 2). Die beiden Lichtspektren 14', 18' werden durch den Bandstoppfilter 22 schärfer voneinander getrennt.

Ferner kann der Bandstoppfilter 22 auch als Anregungsfilter eingesetzt werden, beispielsweise zur Durchführung von Fluoreszenzbildgebung. Das Stoppband kann dann entsprechend gewählt werden, das zur Fluoreszenzbildgebung geeignete Anregungslicht durchzulassen und Licht in angrenzenden Wellenlängenbereichen zu blockieren. Dadurch kann etwa Indocyaningrün (ICG)-Fluoreszenzbildgebung alternativ oder zusätzlich zur multispektralen Bildgebung durchgeführt werden.

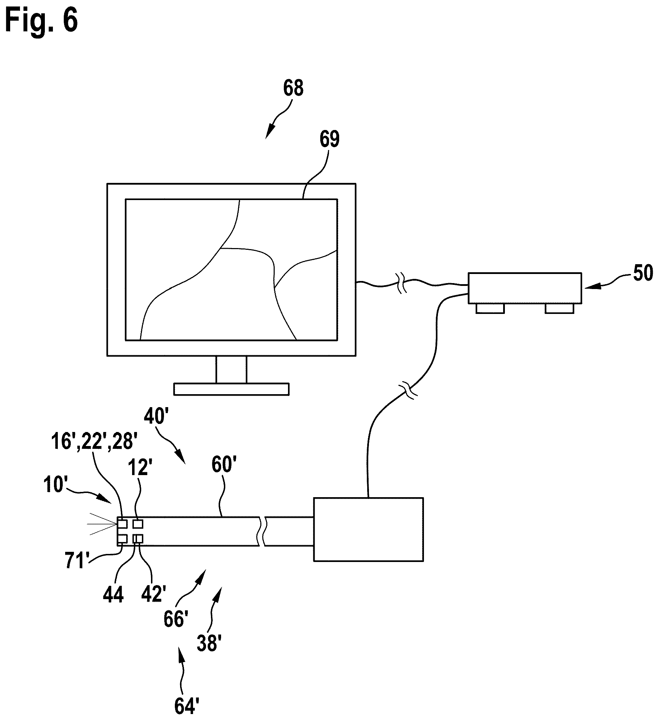

Die Fig. 6 zeigt eine schematische Darstellung einer weiteren Ausführungsform eines medizinischen Bildgebungssystems 38'. Aufgrund der großen Gemeinsamkeiten mit dem Bildgebungssystem 38 wird überwiegend auf Unterschiede eingegangen.

Das medizinische Bildgebungssystems 38' umfasst eine medizinische Lichtquellenvorrichtung 10', eine Bilderfassungseinrichtung 40', welches als Endoskop 66' ausgebildet ist, die Auswerteeinheit 50 und die Anzeigeeinrichtung 68. Bezüglich der Funktionsweise der Auswerteeinheit 50 und der Anzeigeeinrichtung 68 wird auf die obigen Ausführungen verwiesen.

Im Unterschied zu dem in der Fig. 1 gezeigten Ausführungsform ist die Lichtquellenvorrichtung 10' in einem distalen Abschnitt 64' eines Endoskopschafts 60' des Endoskops 66' angeordnet. Die Lichtquellenvorrichtung 10' umfasst ein Beleuchtungsmittel 12', das entsprechend der obigen Ausführungen als rote LED ausgebildet ist. Vor der roten LED ist ein Konvertierungselement 16' angeordnet, das als Phosphorplättchen ausgebildet ist und eine gewisse Transmission aufweist und entsprechend auch als Angleichelement 28' fungiert. Auf dem Phosphorplättchen ist ein Bandstoppfilter 22' angebracht. Hinsichtlich der Funktionsweise dieser Elemente 12', 16', 22', 28' wird auf die obigen Ausführungen verwiesen.

Ebenfalls im distalen Abschnitt 64' ist eine Eingangsoptik 71' und ein Bilderfassungssensor 42' angeordnet. Mittels des Bilderfassungssensors 42' wird multispektrale Bildgebung zur Bestimmung der Oxygenierung durchgeführt. Im Unterschied weist die Bilderfassungseinrichtung 40' lediglich einen Bilderfassungssensor 42' auf, mittels dessen die Bilddaten lichtspektrumsspezifisch erfassbar sind. Dazu ist auf dem Bilderfassungssensor 42' ein Farbfilterarray 44 angeordnet. Dieser ist als herkömmliche Bayer-Matrix ausgebildet.

Bei dieser Ausführungsform wird ausgenutzt, dass die Bayer-Matrix Segmente aufweist, die im sichtbaren Wellenlängenbereich unterschiedlich Licht blockieren, im nahinfraroten Wellenlängenbereich jedoch näherungsweise gleich Licht blockieren bzw. durchlassen.

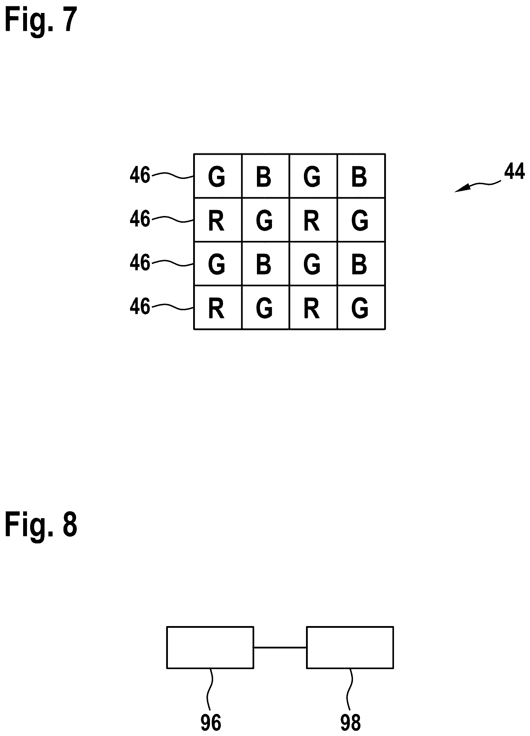

Die Fig. 7 zeigt eine schematische Darstellung des entsprechenden Farbfilterarrays 44. Dieses ist in Segmente 46 unterteil, wobei jeweils vier der Segmente eine Einheit bilden. Die Segmente 46 sind in unterschiedlichen Wellenlängenbereichen des sichtbaren Lichts unterschiedlich lichtdurchlässig. Genauer ist jedes der Segmente 46 als ein Farbfilter ausgebildet und lässt Licht in einer der Grundfarben (Grün, Rot, Blau) durch. Vorliegend sind in einer ersten Zeile abwechselnd Farbfilter zur Transmission grünen Lichts (G) und blauen Lichts (B) vorgesehen. In einer zweiten darauffolgenden Zeile sind abwechselnd Farbfilter zur Transmission roten Lichts (R) und grünen Lichts (G) vorgesehen. Die beiden Zeilen wiederholen sich periodisch auf dem Farbfilterarray 44. Mittels des Bilderfassungssensors 42' werden so einzelne Auslesepunkte ausgelesen, auf die Licht trifft, das durch die einzelnen Segmente getreten ist. In anderen Worten weist der Bilderfassungssensor 42' einzelne Farbkanäle (R, G, B) auf. Diese werden unabhängig voneinander ausgelesen.

Zur multispektralen Bildgebung wird, sofern beispielsweise Beleuchtungslicht mit dem Intensitätsverlauf gemäß Fig. 3 oder Fig. 5 mittels der Lichtquellenvorrichtung 10' bereitgestellt wird, ein roter Farbkanal ausgelesen. Zudem kann vorzugsweise ein blauer Farbkanal ausgelesen werden. Entsprechende Bildinformation, die mittels des blauen Farbkanals ausgelesen wird, basiert lediglich auf Licht im nahinfraroten Wellenlängenbereich; entsprechend basiert sie auf Licht im zweiten Spektralbereich 18'. Die Bildinformation, die mittels des roten Farbkanals ausgelesen wird, basiert hingegen auf Licht im roten und im nahinfraroten Wellenlängenbereich; entsprechend basiert sie auf Licht im ersten und im zweiten Spektralbereich 14', 18'. Um Bildinformation lediglich für den ersten Spektralbereich 14' zu erhalten, wird ein mittels des blauen Farbkanals gemessener Intensitätswert bildpunktweise von dem Intensitätswert gemäß dem roten Farbkanal abgezogen. Dadurch sind die Bilddaten lichtspektrumsspezifisch für das erste Lichtspektrum 14' und das zweite Lichtspektrum 18' mittels lediglich einem Bilderfassungssensor 42' erzeugbar. Basierend auf diesen Bilddaten ist die Auswerteeinheit dazu eingerichtet, den physiologischen Parameter zu bestimmen.

Gemäß weiteren nicht dargestellten Ausführungsformen ist das Konvertierungselement dazu eingerichtet, zeitverzögert das Licht mit dem Ausgangslichtspektrum in das Licht mit dem zweiten Lichtspektrum zu wandeln. Auch dadurch können mittels lediglich einem Bilderfassungssensor Bilddaten lichtspektrumsspezifisch für das erste Lichtspektrum und das zweite Lichtspektrum erzeugt werden. Durch den Zeitverzug kann vor einem Wandeln des Lichts ein Bild aufgenommen werden. In diesem Fall kann vorgesehen sein, dass das Licht im Ausgangslichtspektrum nahezu vollständig gewandelt wird. Dann können die einzelnen Bilder für die Multispektralbildgebung zeitversetzt voneinander erzeugt werden.

Fig. 8 zeigt ein schematische Ablaufdiagramm eines Verfahrens zum Betrieb einer medizinischen Lichtquellenvorrichtung 10, 10' und/oder eines medizinischen Bildgebungssystems 38, 38'. Das Verfahren umfasst den Schritt 96 eines Erzeugens von Licht mit einem Ausgangslichtspektrum 92. Das Verfahren umfasst zudem den Schritt 98 eines Wandelns des Lichts mit dem Ausgangslichtspektrum 92 zumindest teilweise in Licht mit einem zweiten Lichtspektrum 18, 18' derart, dass Beleuchtungslicht gleichzeitig mit Licht mit einem ersten Lichtspektrum 14, 14' und mit dem zweiten Lichtspektrum 18, 18' bereitstellbar ist. Hinsichtlich der Eigenschaften des Beleuchtungslichts und der verschiedenen Lichtspektren wird auf obige Erläuterungen verwiesen.

Im Folgenden sind weitere relevante Aspekte der Erfindung beschrieben. Neben den oben genannten Wellenlängenbereichen bei 660 nm und im Bereich von 940 nm können auch noch weitere Wellenlängenbereiche für multispektrale Bildgebung von Relevanz sein. Beispielsweise bietet es sich für die Darstellung der Oxygenierung des Gewebes an, spektrale Stützstellen im Bereich von Wellenlängen zwischen 540 nm und von 580 nm zu verwenden, da bei diesen Wellenlängen oxygeniertes Hämoglobin zwei prominente Absorptionsmaxima aufweist. Zwischen diesen Wellenlängen weist deoxygeniertes Hämoglobin nur ein Maximum auf. Ebenso bieten sich isosbestische Punkte z.B. bei Wellenlängen von 790nm, 460nm oder 530nm an, an denen die Absorption unabhängig von der Oxygenierung ist. Um nun solche spektrale Stützstellen in einem möglichst kompakten Lichtquellendesign zu kombinieren, bietet es sich an, jeweils einen schmalbandigen, kurzwelligen Bereich mit einem oder mehreren breiten langwelligeren Bereichen zu kombinieren. Es kann dann ein Konvertierungselement, beispielsweise in Form einer Beschichtung auf dem Beleuchtungsmittel bzw. einer LED, verwendet werden, der eine schmalbandige LED und/oder Laserdiode in einen breiteren langwelligeren Bereich konvertiert. Dabei sollten die beiden Bereiche spektral derart voneinander beabstandet sein, dass diese in unterschiedlichen Kanälen des RGGB-Bayer-Pattern oder eines anderen beliebigen Farbfilterarrays detektiert werden.

Beispielsweise können zwei spektral unterschiedliche LEDs, Laserdioden und/oder dergleichen mit Emissionspeaks bei 535nm bzw. bei 555nm verwendet werden, wobei die beiden Laserdioden so beschichtet sind, dass Licht in den Wellenlängenbereich zwischen 650 nm und 700nm bzw. zwischen 850 nm und 950 nm konvertiert wird. Beim Einschalten der 535nm Laserdiode kann somit gleichzeitig eine Stützstelle im Bereich zwischen 650 nm und 700 nm ermittelt werden (Licht im Wellenlängenbereich um 535 nm wird vom grünen Farbkanal bestimmt; Licht im Wellenlängenbereich zwischen 650 nm und 700nm wird vom roten Farbkanal bestimmt). Beim Einschalten der 555 nm Laserdiode kann die Stützstelle im Wellenlängenbereich zwischen 850 nm und 950nm ermittelt werden (Licht im Wellenlängenbereich um 535 nm wird vom grünen Farbkanal bestimmt, Licht im Wellenlängenbereich zwischen 850 nm und 900nm wird von allen Farbkanälen bestimmt). Dadurch können mittels zweier Bilderfassungssensorauslesungsschritte unter Verwendung von zwei Beleuchtungsmitteln vier spektrale Stützstellen erfasst werden.

Neben der Nutzung eines fluoreszenten Farbstoffes zur Erzeugung einer zweiten Wellenlänge bzw. zur Bereitstellung des Konvertierungselements, ist es ebenso denkbar, einen phosphoreszenten Farbstoff auf eine LED bzw. Laserdiode aufzubringen. Durch die zeitverzögerte Aussendung des Phosphoreszenzlichts kann somit durch periodisches Ein- und Ausschalten der LED die Lichtzusammensetzung der Lichtquelle variiert werden. Durch eine synchrone Aufnahme von Bildern kann somit etwa ein erstes Bild mit der gemeinsamen Beleuchtung durch Phosphoreszenzlicht und LED-Licht sowie ein zweites Bild erzeugt werden, welches nur auf der Beleuchtung mittels des Phosphoreszenzlicht beruht.

Mehrere hierin beschriebene Lichtquellenvorrichtungen lassen sich auch kombinieren, um komplexes Beleuchtungslicht bereitstellen zu können, wobei eine einzelne Lichtquellenvorrichtung baulich einfach gehalten werden kann.

### Bezugszeichenliste

- 10: medizinische Lichtquellenvorrichtung
- 12: Beleuchtungsmittel
- 13: optische Achse
- 14: erstes Lichtspektrum
- 15: erster Wellenlängenbereich
- 16: Konvertierungselement
- 18: zweites Lichtspektrum
- 19: zweiter Wellenlängenbereich
- 20: Bandbreite
- 21: Überlappung
- 22: Bandstoppfilter
- 24: spektrales Band
- 26: Hauptwellenlängenbereich
- 28: Angleichelement
- 30: Intensität
- 32: Intensität
- 34: weiteres Beleuchtungsmittel
- 35: optische Achse
- 36: weiteres Konvertierungselement
- 38: medizinisches Bildgebungssystem
- 40: Bilderfassungseinrichtung
- 42: Bilderfassungssensor
- 43: Strahlteiler
- 44: Farbfilterarray
- 46: Segment
- 48: optischer Filter
- 50: Auswerteeinheit
- 52: erste spektrale Stützstelle
- 54: zweite spektrale Stützstelle
- 56: erstes Intensitätsmaximum
- 58: zweites Intensitätsmaximum
- 60: Endoskopschaft
- 62: proximaler Abschnitt
- 64: distaler Abschnitt
- 66: Endoskop
- 68: Anzeigeeinrichtung
- 69: Darstellung
- 70: Lichtleiter
- 71: Eingangsoptik
- 72: Beleuchtungsvorrichtung
- 73: Kabel
- 74: Gehäuse
- 75: Kühlvorrichtung
- 76: Strahlkombinierer
- 77: gemeinsamer optischer Pfad
- 78: Einschiebevorrichtung
- 80: optische Schnittstelle
- 81: Innenraum
- 82: Intensitätsverlauf
- 83: Abszisse
- 84: Ordinate
- 85: Abszisse
- 86: Ordinate
- 87: Kurve
- 88: Kurve
- 89: Umkehrpunkt
- 92: Ausgangslichtspektrum
- 93: Ausgangswellenlängenbereich
- 96: Schritt
- 98: Schritt

## Patentansprüche

1. Medizinische Lichtquellenvorrichtung (10), umfassend:
- ein Beleuchtungsmittel (12), welches dazu eingerichtet ist, Licht mit einem Ausgangslichtspektrum (92) zu erzeugen, und
- ein Konvertierungselement (16), welches dazu eingerichtet ist, das Licht mit dem Ausgangslichtspektrum (92) zumindest teilweise in Licht mit einem zweiten Lichtspektrum (18) derart zu wandeln, dass Beleuchtungslicht gleichzeitig mit Licht mit einem ersten Lichtspektrum (14) und mit dem zweiten Lichtspektrum (18) bereitstellbar ist,
wobei das erste Lichtspektrum (14) spektral dem Ausgangslichtspektrum (92) entspricht,
wobei das erste Lichtspektrum (14) zumindest teilweise in einem kurzwelligeren Wellenlängenbereich als das zweite Lichtspektrum (18) liegt,
wobei das erste Lichtspektrum (14) das zweite Lichtspektrum (18) höchstens zu 50 % überlappt,
wobei das erste Lichtspektrum (14) im Vergleich zu dem zweiten Lichtspektrum (18) eine geringere Bandbreite (20) aufweist, und
wobei anhand des ersten Lichtspektrums (14) und des zweiten Lichtspektrums (18) wenigstens ein physiologischer Parameter bestimmbar ist.

2. Medizinische Lichtquellenvorrichtung (10) nach Anspruch 1,
wobei das Beleuchtungsmittel (12) dazu eingerichtet ist, rotes Licht zu erzeugen und das Konvertierungselement (16) dazu eingerichtet ist, rotes Licht in nahinfrarotes Licht zu wandeln.

3. Medizinische Lichtquellenvorrichtung (10) nach Anspruch 1 oder 2, ferner umfassend:
- einen Bandstoppfilter (22) zum Blockieren von Licht in einem spektralen Band (24), welches spektral zwischen Hauptwellenlängenbereichen (26) des ersten Lichtspektrums (14) und des zweiten Lichtspektrums (18) liegt.

4. Medizinische Lichtquellenvorrichtung (10) nach einem der vorhergehenden Ansprüche, ferner umfassend:
- ein Angleichelement (28), welches dazu eingerichtet ist, eine Intensität (30) des Lichts mit dem ersten Lichtspektrum (14) und eine Intensität (32) des Lichts mit dem zweiten Lichtspektrum (18) anzugleichen.

5. Medizinische Lichtquellenvorrichtung (10) nach Anspruch 4,
wobei das Angleichelement (28) durch das Konvertierungselement (16) ausgebildet ist, und
wobei eine Transmission des Konvertierungselements (16) derart gewählt ist, dass die Intensität (30) des Lichts mit dem ersten Lichtspektrum (14) und die Intensität (32) des Lichts mit dem zweiten Lichtspektrum (18) angeglichen bereitstellbar sind.

6. Medizinische Lichtquellenvorrichtung (10) nach einem der vorhergehenden Ansprüche, ferner umfassend:
- ein weiteres Beleuchtungsmittel (34) und ein weiteres Konvertierungselement (36), welche andere spektrale Eigenschaften als das Beleuchtungsmittel (12) und das Konvertierungselement (16) aufweisen.

7. Medizinische Lichtquellenvorrichtung (10) nach einem der vorhergehenden Ansprüche,
wobei das Konvertierungselement (16) dazu eingerichtet ist, zeitverzögert das Licht mit dem Ausgangslichtspektrum (92) in das Licht mit dem zweiten Lichtspektrum (18) zu wandeln.

8. Medizinisches Bildgebungssystem (38), insbesondere endoskopisches, mikroskopisches und/oder exoskopisches Bildgebungssystem, umfassend:
- eine medizinische Lichtquellenvorrichtung (10) nach einem der vorhergehenden Ansprüche, welche dazu eingerichtet ist, Beleuchtungslicht zur Beleuchtung eines Objektbereichs bereitzustellen, und
- eine Bilderfassungseinrichtung (40), welche dazu eingerichtet ist, den Objektbereich abzubilden und Bilddaten des Objektbereichs zu erzeugen, wobei die Bilddaten räumliche und spektrale Informationen umfassen, welche sich auf eine Rückstrahlung des Objektbereichs in Folge der Beleuchtung mit Licht mit dem ersten Lichtspektrum (14) und dem zweiten Lichtspektrum (18) beziehen.

9. Medizinisches Bildgebungssystem (38) nach Anspruch 8,
wobei die Bilddaten lichtspektrumsspezifisch für das erste Lichtspektrum (14) und das zweite Lichtspektrum (18) erzeugbar sind.

10. Medizinisches Bildgebungssystem (38) nach Anspruch 8 oder 9,
wobei die Bilderfassungseinrichtung (40) zwei Bilderfassungssensoren (42) umfasst, welche in unterschiedlichen Wellenlängenbereichen lichtempfindlich sind.

11. Medizinisches Bildgebungssystem (38) nach Anspruch 9,
wobei die Bildererfassungseinrichtung (40) einen Bilderfassungssensor (42) umfasst, der dazu eingerichtet ist, die Bilddaten für das erste Lichtspektrum (14) und das zweite Lichtspektrum (18) zu erfassen.

12. Medizinisches Bildgebungssystem (38) nach Anspruch 11,
wobei der Bilderfassungssensor (42) einen Farbfilterarray (44) umfasst, welcher Segmente (46) umfasst, welche in unterschiedlichen Wellenlängenbereichen des sichtbaren Lichts unterschiedlich lichtdurchlässig sind, wobei die Segmente (46) zumindest in einem nahinfraroten Wellenlängenbereich zumindest annähernd gleich lichtdurchlässig sind.

13. Medizinisches Bildgebungssystem (38) nach einem der Ansprüche 8 bis 12,
wobei die Bilderfassungseinrichtung (40) einen optischen Filter (48) umfasst, der dazu eingerichtet ist, Licht mit dem ersten Lichtspektrum (14) zu blockieren.

14. Medizinisches Bildgebungssystem (38) nach einem der Ansprüche 8 bis 13, ferner umfassend:
- eine Auswerteeinheit (50), die dazu eingerichtet ist, nach Maßgabe der räumlichen und spektralen Informationen den physiologischen Parameter zu bestimmen.

15. Medizinisches Bildgebungssystem (38) nach Anspruch 14,
wobei die Auswerteeinheit (50) dazu eingerichtet ist, zumindest zwei unterschiedliche Intensitätswerte miteinander zu vergleichen, die sich auf das erste Lichtspektrum (14) und das zweite Lichtspektrum (18) beziehen, um den physiologischen Parameter zu bestimmen.

16. Medizinisches Bildgebungssystem (38) nach Anspruch 14 oder 15,
wobei die Auswerteeinheit (50) dazu eingerichtet ist, den physiologischen Parameter anhand wenigstens zweier unterschiedlicher spektraler Stützstellen (52, 54) zu bestimmen, wobei eine erste spektrale Stützstelle (52) im Bereich eines ersten Intensitätsmaximums (56) des ersten Lichtspektrums (14) und eine zweite spektrale Stützstelle (54) im Bereich eines zweiten Intensitätsmaximums (58) des zweiten Lichtspektrums (18) liegt.

17. Medizinisches Bildgebungssystem (38) zumindest nach einem der Ansprüche 8 bis 16, ferner umfassend:
- einen Endoskopschaft (60) mit einem proximalen Abschnitt (62) und einem distalen Abschnitt (64), wobei die Lichtquellenvorrichtung (10) im distalen Abschnitt (64) angeordnet ist.

18. Verfahren zum Betrieb einer medizinischen Lichtquellenvorrichtung (10), insbesondere nach einem der Ansprüche 1 bis 7, und/oder eines medizinischen Bildgebungssystems, insbesondere nach einem der Ansprüche 8 bis 17, umfassend die folgenden Schritte:
- Erzeugen von Licht mit einem Ausgangslichtspektrum (92); und
- Wandeln des Lichts mit dem Ausgangslichtspektrum (92) zumindest teilweise in Licht mit einem zweiten Lichtspektrum (18) derart, dass Beleuchtungslicht gleichzeitig mit Licht mit einem ersten Lichtspektrum (14) und mit dem zweiten Lichtspektrum (18) bereitstellbar ist,
wobei das erste Lichtspektrum (14) spektral dem Ausgangslichtspektrum (92) entspricht,
wobei das erste Lichtspektrum (14) zumindest teilweise in einem kurzwelligeren Wellenlängenbereich als das zweite Lichtspektrum (18) liegt,
wobei das erste Lichtspektrum (14) das zweite Lichtspektrum (18) höchstens zu 50 % überlappt,
wobei das erste Lichtspektrum (14) im Vergleich zu dem zweiten Lichtspektrum (18) eine geringere Bandbreite (20) aufweist, und
wobei anhand des ersten Lichtspektrums (14) und des zweiten Lichtspektrums (18) wenigstens ein physiologischer Parameter bestimmbar ist.
